# EUROPEAN PATENT APPLICATION

(11) **EP 3 561 644 A1**
(43) Date of publication of application: **30.10.2019**
(21) Application number: 18169722.8
(22) Date of filing: 27.04.2018
(51) Int. Cl.: G06F 3/01, G06F 3/16, A61B 5/024, A61B 5/11, A61B 5/18, A61B 5/00, B60W 40/08

(54) **MULTIMEDIA EFFECT BASED ON PHYSIOLOGICAL DATA OF A USER OF A VEHICLE**

(71) Applicant: Ningbo Geely Automobile Research & Development Co. Ltd., Hangzhou Bay New District Ningbo 315336 (CN)
(72) Inventor: DOMBROVSKIS, Sergejs, 422 57 HISINGS BACKA (SE)
(74) Representative: Zacco Sweden AB

(57) **Abstract**

A method and a user interface system (100) in a vehicle for creating an emotional bond between a person (1) and the vehicle. The user interface system (100) comprising a sensor (10a, 10b, 10c, 10d,..., 10n) configured to detect a real-time physiological data of a person (1), a multimedia interface (50a, 50b, 50c, 50d, ..., 50n) configured to output multimedia and a processing circuitry (102). The processing circuitry (102) is configured to cause the user interface system (100) to detect, by the sensor (10a, 10b, 10c, 10d,..., 10n), a real-time physiological data of a person (1); generate a multimedia effect based on the real-time physiological data; and output the multimedia effect to the person (1) via a multimedia interface (50a, 50b, 50c, 50d, ..., 50n) of the device.

## Description

### TECHNICAL FIELD

The disclosure pertains to the field of interaction between a person and a device.

### BACKGROUND

Today human persons are interacting with plurality of devices. The space where interactions between persons and machines occur is sometimes referred to as a "man-machine interface" or when the machine in question is a computer a "human-computer interface". In the interaction between a person and a device one of the goals is to allow an effective operation and control of the device from the person, and also for the device to feedback information to the person interacting with the device. Persons are interacting with all kinds of devices such as computers, smartphones, process machines, tools, buildings and vehicles etc. In the interaction between the person and the device, the person may input information to the device via an input interface, e.g. via a key, a switch, a button, a keyboard, a touch sensitive control, a voice operated control, a gesture recognition device etc. The person interacting with the device can get output information from the device via an output interface such as a graphical user interface, a light source, a sound source, a tactile feedback etc. In the space between the person and the device, the interaction is often designed to be intuitive, reliable, and easy to understand for the person in order to close the distance between the person and the device. Operation of the device is often desired to be natural and comfortable for the person in order to bring the device closer to the person.

### SUMMARY

Today there is a demand for a better interaction between a person and the device that the person is interacting with. The inventor has realized that in order to bring the device closer to the person, there is a need for a common linkage effect that connects the person with the device in a natural way so that the person can recognize an effect that occurs both in the device and in the person. The inventor has identified a way to form an emotional bond between the user and the device by mirroring an effect of the person's body with an effect of the device that the person is interacting with. According to an aspect the inventor has realized that mirroring e.g. a heartbeat or breathing effect of the person with an output effect of the device, an emotional bond can be formed between the person and the device. The inventor has realized that this kind of mirroring effect contributes to a user satisfaction and not only creates an emotional bond between the person and the device but also increases the value of the device to the person. According to an aspect the invention is a method which use heartbeat sensing data to control a wide variety of multimedia content, as output information from the device.

The disclosure proposes a method of generating a multimedia effect for creating an emotional bond between a person and a device. The method comprising detecting, by a sensor, a real-time physiological data of a person. The method is followed by the step of generating a multimedia effect based on the real-time physiological data and followed by the step of outputting the multimedia effect to the person via a multimedia interface of the device. An advantage with the method is that the method is generating a common linkage effect that connects the person with the device in a natural way.

According to an aspect the method further comprising the step of adjusting the multimedia effect continuously in real-time based on a change in the detected real-time physiological data. This means that the multimedia effect is constantly mirroring the person's physiological data.

According to an aspect the method further comprising the step of simulating a multimedia effect based on previous real-time physiological data. In other words, the multimedia effect can be maintained similar as the last detected real-time physiological data. Hence, the multimedia effect may be simulated in order to maintain the common linkage between the person and the device even if the detection of the real-time physiological data of the person is absent.

According to an aspect the method further comprising the step of generating a control signal based on the real-time physiological data and a modification data and the step of generating the multimedia effect based on the real-time physiological data and the control signal. This means that the multimedia effect can be generated not only based on the real-time physiological data but also based on a modification signal that affects the generation of the multimedia effect so that both the real-time physiological data and the modification signal are used as input for the generation of the multimedia effect.

According to an aspect the method further comprising the step of transitioning the simulated multimedia effect into a real-time multimedia effect based on a detected real-time physiological data. This means that e.g. in the case that the detection of the real-time physiological data has been absent but is re-established, then the simulation of the multimedia effect can be ceased and instead the real-time physiological data resumes to be the input for generating the multimedia effect.

According to an aspect the multimedia effect is at least one of a visual stimulus effect; a haptic stimulus effect; or an audio stimulus effect. In other words, the multimedia effect can be observed using at least one of the person's eyes, feel or ears.

According to an aspect the multimedia interface is at least one of a visual stimulus device, a haptic stimulus device or an audio stimulus device. In other words, a plurality of different devices can be used for establishing the linkage effect that connects the person with the device in a natural way.

According to an aspect the physiological data is at least one of heartbeat data; breathing data; eye movement data; eye blinking data; eye pupil size data; eye gaze data; skin conductance data; muscle tension data or body temperature data. This means that a plural of physiological data of the person can be used for generating the multimedia effect.

According to an aspect the physiological data is breathing data and the multimedia effect is a pulsing light that is outputted via a light source configured to alternate an intensity and/or a frequency of the light for visualizing the actual breathing of the person. This means that the person can see the multimedia effect by observing the pulsing light and recognize the multimedia effect as the common linkage effect that connects the person with the device in a natural way.

According to an aspect the sensor is any of a heartbeat sensor for measuring the pulse of the person; a respiration sensor for measuring the breathing of the person; a camera for capturing images and video of the person; a microphone for recording sound of the person; or a pressure sensor for measuring a force generated by the person. In other words, different sensors can be used for detecting the real-time physiological data of a person.

The disclosure further proposes a user interface system in a vehicle for creating an emotional bond between a person and the vehicle. The user interface system comprising a sensor configured to detect a real-time physiological data of a person, a multimedia interface configured to output multimedia and a processing circuitry. The processing circuitry is configured to cause the user interface system to detect, by a sensor, a real-time physiological data of a person. The processing circuitry is further configured to generate a multimedia effect based on the real-time physiological data and output the multimedia effect to the person via a multimedia interface of the device. An advantage with the method is that the method is generating a common linkage effect that connects the person with the device in a natural way.

According to an aspect the processing circuitry is further configured to cause the user interface system to adjust the multimedia effect continuously in real-time based on a change in the detected real-time physiological data. This means that the multimedia effect is constantly mirroring the person's physiological data.

According to an aspect the processing circuitry is further configured to cause the user interface system to simulate a multimedia effect based on previous real-time physiological data. In other words, the multimedia effect can be maintained similar as the last detected real-time physiological data. Hence, the multimedia effect may be simulated in order to maintain the common linkage between the person and the device even if the detection of the real-time physiological data of the person is absent.

According to an aspect the processing circuitry is further configured to cause the user interface system to generate a control signal based on the real-time physiological data and a modification data and generate a multimedia effect based on the real-time physiological data and the control signal. This means that the multimedia effect can be generated not only based on the real-time physiological data but also based on a modification signal that affects the generation of the multimedia effect so that both the real-time physiological data and the modification signal are used as input for the generation of the multimedia effect.

According to an aspect the visual stimulus device is any of a display configured to display a graphical user interface; or a light source configured to alternate illumination of light by changing any of an intensity, a color or a frequency of the light. In other words, a plurality of different visual stimulus devices can be used for establishing the linkage effect that connects the person with the device in a natural way.

### BRIEF DESCRIPTION OF THE DRAWINGS

The foregoing will be apparent from the following more particular description of the example embodiments, as illustrated in the accompanying drawings in which like reference characters refer to the same parts throughout the different views. The drawings are not necessarily to scale, emphasis instead being placed upon illustrating the example embodiments.
Figure 1 illustrates an example of the proposed user interface system in a vehicle.
Figure 2 illustrates an illustrative overview of the method steps according to an aspect of the disclosure.
Figure 3 illustrates a flow chart of the method steps according to an aspect of the disclosure.

### DETAILED DESCRIPTION

Aspects of the present disclosure will be described more fully hereinafter with reference to the accompanying drawings. The method and system disclosed herein can, however, be realized in many different forms and should not be construed as being limited to the aspects set forth herein. Like numbers in the drawings refer to like elements throughout.

The terminology used herein is for the purpose of describing particular aspects of the disclosure only, and is not intended to limit the disclosure.

In some implementations and according to some aspects of the disclosure, the functions or steps noted in the blocks can occur out of the order noted in the operational illustrations. For example, two blocks shown in succession can in fact be executed substantially concurrently or the blocks can sometimes be executed in the reverse order, depending upon the functionality/acts involved.

In the drawings and specification, there have been disclosed exemplary aspects of the disclosure. However, many variations and modifications can be made to these aspects without substantially departing from the principles of the present disclosure. Thus, the disclosure should be regarded as illustrative rather than restrictive, and not as being limited to the particular aspects discussed above. Accordingly, although specific terms are employed, they are used in a generic and descriptive sense only and not for purposes of limitation.

It should be noted that the word "comprising" does not necessarily exclude the presence of other elements or steps than those listed and the words "a" or "an" preceding an element do not exclude the presence of a plurality of such elements. It should further be noted that any reference signs do not limit the scope of the claims, that the example embodiments may be implemented at least in part by means of both hardware and software, and that several "means", "units" or "devices" may be represented by the same item of hardware.

Today persons are interacting with a plurality of devices. The space where interactions between persons and devices occur is sometimes referred to as a "man-machine interface" or when the device in question is a computer it is sometimes referred to as a "human-computer interface". In the interaction between a person and a device one of the goals is to allow an effective operation and control of the device from the person, and also for the device to feedback information to the person interacting with the device. Persons are interacting with a plurality of devices such as computers, smartphones, process machines, tools, buildings and vehicles etc. In the interaction between the person and the device, the person may input information to the device via an input interface, e.g. via a key, a switch, a button, a keyboard, a touch sensitive control, a voice operated control, a gesture recognition device etc. The person interacting with the device can get output information from the device via an output interface such as a graphical user interface, a light source, a sound source, a tactile feedback etc.

There is a demand for a better interaction between a person and the device that the person is interacting with. The inventor has realized that in order to bring the device closer to the person, there is a need for a common linkage effect that connects the person with the device in a natural way so that the person can recognize an effect that occurs both in the device and in the person.

The inventor has identified a way to form an emotional bond between the user and the device by mirroring an effect of the person's body with an effect of the device that the person is interacting with. According to an aspect the inventor has realized that mirroring e.g. a heartbeat or a breathing effect of the person with an output effect of the device, an emotional bond can be formed between the person and the device.

The inventor has realized that this kind of mirroring effect contributes to a user satisfaction and not only creates an emotional bond between the person and the device but also increases the value of the device to the person. According to an aspect the invention is a method which use heartbeat sensing data to control a wide variety of multimedia content, as output information from the device.

The disclosure proposes a method of generating a multimedia effect for creating an emotional bond between a person and a device. According to an aspect the method can be applied on a wide range of different devices.

The inventor has seen a particular use for the invention when the device is a vehicle. In addition to the method of generating a multimedia effect, the disclosure further proposes a user interface system 100 in a vehicle for creating an emotional bond between a person 1 and the vehicle.

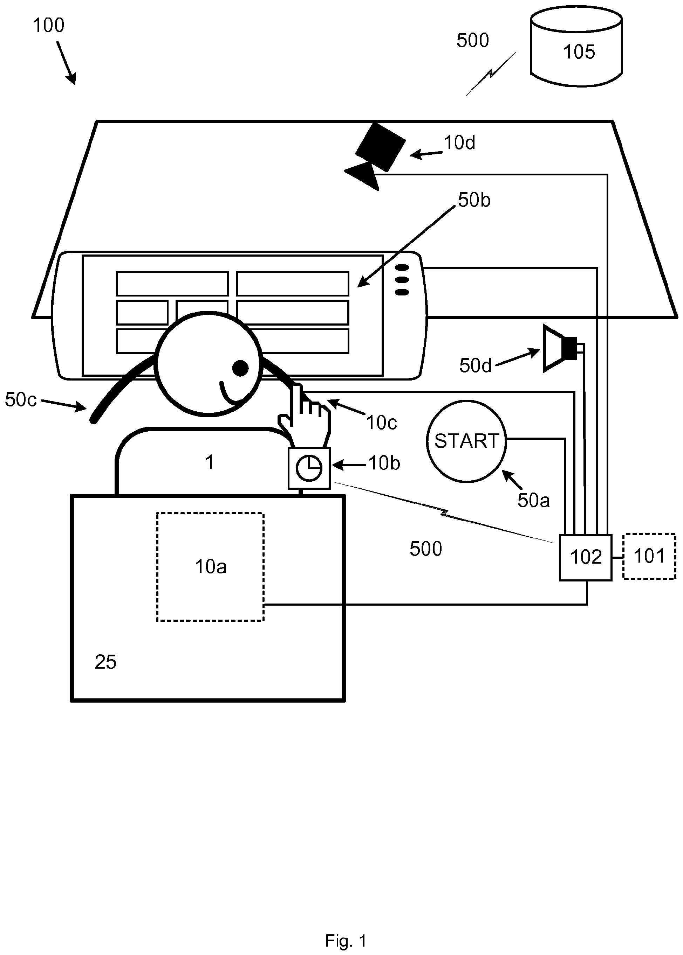

As illustrated in Fig 1, the user interface system 100 comprising a sensor 10a, 10b, 10c, 10d,..., 10n configured to detect a real-time physiological data of a person 1, a multimedia interface 50a, 50b, 50c, 50d, ..., 50n configured to output multimedia, and a processing circuitry 102. The processing circuitry 102 is configured to cause the user interface system 100 to detect, by the sensor 10a, 10b, 10c, 10d,..., 10n, a real-time physiological data of a person 1. The processing circuitry 102 is further configured to generate a multimedia effect based on the real-time physiological data and output the multimedia effect to the person 1 via a multimedia interface 50a, 50b, 50c, 50d, ..., 50n of the device. An advantage with the method is that the method is generating a common linkage effect that connects the person 1 with the device in a natural way.

According to an aspect the sensor 10a, 10b, 10c, 10d,..., 10n configured to detect a real-time physiological data of a person 1. The term real-time refers to the actual time during when the detection occurs. In the user interface system 100 real-time is dependent on the speed and capacity of the sensor 10a, 10b, 10c, 10d,...,10n and the processing circuitry 102. Real-time is normally in the order of milliseconds, and sometimes in the order of microseconds, but can be faster and slower.

According to an aspect the sensor 10a, 10b, 10c, 10d,..., 10n is any of a heartbeat sensor for measuring the pulse of the person 1; a respiration sensor for measuring the breathing of the person 1; a camera for capturing images and video of the person 1; a microphone for recording sound of the person 1; or a pressure sensor for measuring a force generated by the person 1. In other words, different sensors can be used for detecting the real-time physiological data of a person 1.

In the Fig 1 a number of example sensors 10a, 10b, 10c, and 10d are visualized. According to an aspect the sensor 10a in Fig 1 is a respiration sensor. It is known that some portion of the human upper body always expands with respiratory movement, and this phenomena makes the backrest of the vehicle seat 25 an ideal place to measure respiration using pressure sensors. According to an aspect the sensor 10b in Fig 1 is a heartbeat sensor that is worn on the arm of the person 1 in the vehicle, and wirelessly connected to the processing circuitry 102. Heartbeat sensors are commonly integrated in e.g. smartwatches and other wearable devices for e.g. sports and health care purposes.

According to an aspect the sensor 10c in Fig 1 is a pressure sensor integrated in the steering wheel for measuring the force generated by the muscles in the hands of the person 1 in the vehicle. According to an aspect the sensor 10d in Fig 1 is a camera for measuring e.g. the pupil size of the person 1 in the vehicle.

The sensor 10a, 10b, 10c, 10d,...,10n can be used for other purposes when not used in the user interface system 100. According to an aspect the sensor 10a, 10b, 10c, 10d,...,10n is any existing sensor installed on or in the vehicle primarily used for other purposes. According to an aspect the sensor 10a, 10b, 10c, 10d is any of a humidity sensor or a temperature sensor installed in the vehicle for controlling the climate in the vehicle. According to an aspect the sensor 10a, 10b, 10c, 10d,...,10n is a camera installed in the vehicle for security or control purposes.

According to an aspect the physiological data is at least one of heartbeat data; breathing data; eye movement data; eye blinking data; eye pupil size data; eye gaze data; skin conductance data; muscle tension data or body temperature data. This means that a plural of physiological data of the person 1 can be used for generating the multimedia effect.

According to an aspect the multimedia interface 50a, 50b, 50c, 50d, ..., 50n is at least one of a visual stimulus device; a haptic stimulus device or an audio stimulus device. In other words, a plurality of different devices can be used for establishing the linkage effect that connects the person 1 with the device in a natural way. According to an aspect the visual stimulus device is a display configured to display a graphical user interface. The display is for example any of a speedometer, a dashboard control or a touch sensitive display of a vehicle. In an example a graphical user interface of a speedometer is changing shape, color and size dependent on the breathing of the person 1 for generating a user interface that is mirroring the actual breathing of the person 1.

According to an aspect the visual stimulus device is a light source. In one example a light source is configured to alternate the illumination of light by changing any of an intensity, a colour or a frequency of the light.

According to an aspect the physiological data is breathing data and the multimedia effect is a pulsing light that is outputted via a light source configured to alternate an intensity and/or a frequency of the light for visualizing the actual breathing of the person 1. According to an aspect the light source is integrated in a button configured to start the vehicle. In other words, the person 1 in the vehicle can see the multimedia effect by observing the pulsing light and recognize the multimedia effect as the common linkage effect that connects the person 1 with the device in a natural way.

According to an aspect the visual stimulus device is a physical device configured to change at least one of the shape, size, colour or transparency of the physical device. According to an aspect the visual stimulus device is designed using any of smart plastic or a memory metal. In one example the visual stimulus device is the knob of the gear lever in a vehicle configured to change size dependent on the pulse of the person 1.

According to an aspect the haptic stimulus device is a steering wheel configured to alternate an intensity or a frequency of a vibration of the steering wheel. In one example the vibration of the steering wheel is dependent on the pulse of the person 1. In one example the vibration is caused by any of a vibrator, a piezo electric element, a smart metal or a smart plastic. According to an aspect the haptic stimulus device is a steering wheel configured to alternate a resistance in the steering of the steering wheel.

According to an aspect the haptic stimulus device is an inflatable seat that is configured to squeeze or massage the person 1 sitting in the seat. According to an aspect the haptic stimulus device is a window that is configured to change its transparency using a dimmable glass controlled by electricity.

According to an aspect the audio stimulus device is any of a speaker, a buzzer, a sound system. In one example the speaker is a dedicated speaker for a button in form of a small speaker that is inside or adjacent to the button.

According to an aspect the visual stimulus device is any of: a display configured to display a graphical user interface; or a light source configured to alternate illumination of light by changing any of an intensity, a color or a frequency of the light. In other words, a plurality of different visual stimulus devices can be used for establishing the linkage effect that connects the person 1 with the device in a natural way.

In the Fig 1 a number of example multimedia interfaces 50a, 50b, 50c and 50d are visualized. According to an aspect the multimedia interface 50a is a button configured to start the vehicle. According to an aspect the button is configured to change its light intensity with a certain frequency for generating a multimedia effect that is mirroring the breathing of the person 1 in the vehicle.

According to an aspect the multimedia interface 50b is a display. The display is for example any of a speedometer, a dashboard control or a touch sensitive display of the vehicle. According to an aspect the multimedia interface 50c is a vibrator. The vibrator is for example integrated in the steering wheel of the vehicle and configured for giving the person 1 in the vehicle a tactile feedback when holding the steering wheel. According to an aspect the multimedia interface 50d is a speaker. The speaker is for example integrated in a vehicle sound system.

The user interface system 100 further comprise a processing circuitry 102 as illustrated in Fig 1. According to an aspect the user interface system 100 further comprise a memory 101 configured to store information.

According to an aspect at least one sensor 10a, 10b, 10c, 10d,..., 10n and/or at least one multimedia interface 50a, 50b, 50c, 50d, ..., 50n is arranged to communicate wirelessly with the processing circuitry 102 via a communication network 500. In one example the communication network 500 is a standardized wireless local area network such as a Wireless Local Area Network, WLAN, Bluetooth™, ZigBee, Ultra-Wideband, Near Field Communication, NFC, Radio Frequency Identification, RFID, or similar network. In one example the communication network is a standardized wireless wide area network such as a Global System for Mobile Communications, GSM, Extended GSM, General Packet Radio Service, GPRS, Enhanced Data Rates for GSM Evolution, EDGE, Wideband Code Division Multiple Access, WCDMA, Long Term Evolution, LTE, Narrowband-loT, 5G, Worldwide Interoperability for Microwave Access, WiMAX or Ultra Mobile Broadband, UMB or similar network. The communication network 500 can also be a combination of both a local area network and a wide area network. The communication network 500 can also be a wired network. According to an aspect of the disclosure the communication network 500 is defined by common Internet Protocols.

According to an aspect user interface system 100 comprise a memory 101 and processing circuitry 102 that are at a remote location 105 as illustrated in Fig 1. According to an aspect the remote location is a server 105 that is wirelessly connected to the user interface system 100 via a communication network 500.

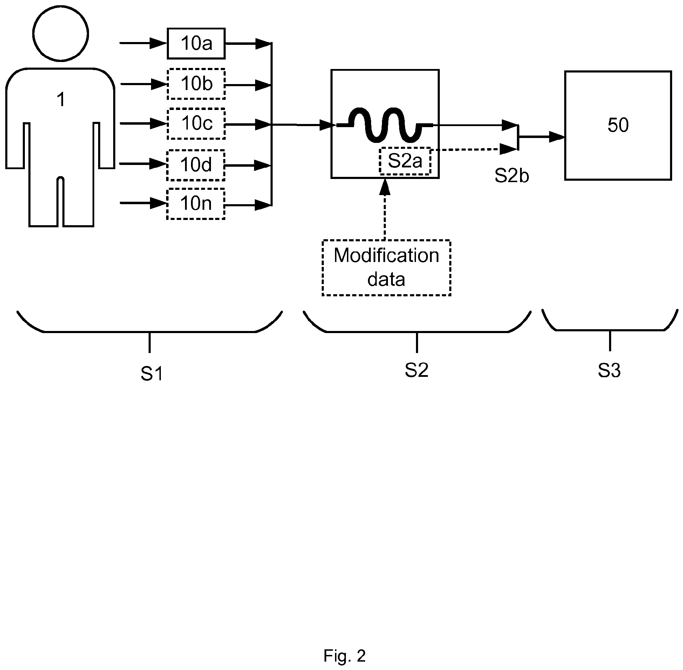

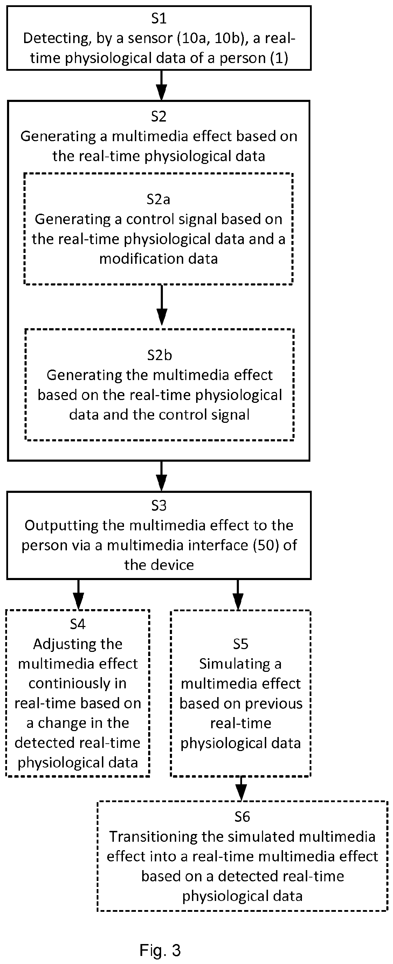

The disclosure further proposes a method of generating a multimedia effect for creating an emotional bond between a person 1 and a device that will be described with the illustrations in Fig 2 and Fig 3. Fig 2 illustrates an illustrative overview of the method steps according to an aspect of the disclosure. Fig 3 illustrates a flow chart of the method steps according to an aspect of the disclosure.

Fig 2 illustrates the detecting of the sensor 10a, 10b, 10c, 10d,..., 10n, that normally occurs close to the person 1, that is referring to step S1 of the method. According to an aspect, as illustrated in Fig 2, the detecting comprising detecting a real-time physiological data by two or more sensors 10a, 10b, 10c, 10d,..., 10n that are combined into one average real-time physiological data for generating the multimedia effect referring to step S2 of the method. In one example, plural of heartbeat sensors are combined to produce one heartbeat physiological data. In one example the heartbeat sensors are located in e.g. the smartwatch worn by the person 1, in the steering wheel for measuring the pulse in the hand of the person 1. A camera may further analyze the fluctuation in color of the skin for determining the heartbeat of the person 1. The different measured heartbeats are compared and one heartbeat is represented as the real-time physiological data of the person 1 that is referring to step S1 of the method, as illustrated in the Fig 2.

According to an aspect one of plural sensors used for detecting a real-time physiological data is detecting data that is inaccurate. According to an aspect plural real-time physiological data are compared by the processing circuitry 102 in order to filter out any inaccurate real-time physiological data. In one example, the sensors 10a, 10b, 10c and 10n in Fig 2 are all detecting very similar real-time physiological data while sensor 10 d in Fig 2 is detecting a real-time physiological data that deviates from the real-time physiological data from the sensors 10a, 10b, 10c and 10n. This information is the used by the processing circuitry 102 to filter out the real-time physiological data from the sensor 10 d.

As illustrated in Fig 2, according to an aspect the step S2 of generating a multimedia effect based on the real-time physiological data further comprise the step S2a of generating a control signal based on the real-time physiological data and a modification data and the step S2b of generating the multimedia effect based on the real-time physiological data and the control signal that will be described in more detail below. According to an aspect the real-time physiological data is described by a mathematical function. In Fig 2 the real-time physiological data is illustrated by a function visualized as a sinus curve. According to an aspect the modification data is manipulating the function, illustrated as the sinus curve, of the real-time physiological data so that the control signal is different from the real-time physiological data. According to an aspect the modification data is changing the amplitude or the frequency of the function describing the real-time physiological data. Fig 2 illustrates the step S3 of outputting the multimedia effect to the person 1 via a multimedia interface 50 of the device.

The method is now described with reference to the illustrated flow chart of Fig 3. The method comprising the step S1 of detecting, by a sensor 10a, 10b, 10c, 10d,..., 10n, a real-time physiological data of a person 1. The method is followed by the step S2 of generating a multimedia effect based on the real-time physiological data and followed by the step S3 of outputting the multimedia effect to the person 1 via a multimedia interface 50a, 50b, 50c, 50d, ..., 50n of the device. An advantage with the method is that the method is generating a common linkage effect that connects the person 1 with the device in a natural way.

According to an aspect the method further comprising the step S4 of adjusting the multimedia effect continuously in real-time based on a change in the detected real-time physiological data. This means that the multimedia effect is constantly mirroring the person's physiological data. In other words, the multimedia effect based on the real-time physiological data of the person 1 is adapting and is constantly changing according to a direct change of the real-time physiological data of the person 1. In one example, the heartbeat of the person 1 interacting with the device is at a higher pace in the beginning of the interactive session, but getting lower after a certain time, and the change of the pulse is mirrored in the multimedia effect of the multimedia interface 50. If the heartbeat of the person 1 is increasing for some reason, the increase of the heartbeat is also mirrored in the multimedia effect of the multimedia interface 50.

According to an aspect the method further comprising the step S5 of simulating a multimedia effect based on previous real-time physiological data. In other words, the multimedia effect can be maintained similar as the last detected real-time physiological data. Hence, the multimedia effect may be simulated in order to maintain the common linkage between the person 1 and the device even if the detection of the real-time physiological data of the person 1 is absent.

According to an aspect the simulation if the multimedia effect is extrapolated based on previous real-time physiological data. In one example the previous real-time physiological data is a heartrate data that is slowly decreasing at a certain rate. According to an aspect the simulated multimedia effect is mirroring the decreasing heartrate at the same rate. In one example the real-time physiological data is temporarily inaccurate, with a sudden high or low heartbeat, wherein the simulating of the multimedia effect is immediately started trigged by that the heartbeat data is above or below a predefined threshold for the heartbeat of the person 1.

According to an aspect the method further comprising the step S2a of generating a control signal based on the real-time physiological data and a modification data and the step S2b of generating the multimedia effect based on the real-time physiological data and the control signal. This means that the multimedia effect can be generated not only based on the real-time physiological data but also based on a modification signal that affects the generation of the multimedia effect so that both the real-time physiological data and the modification signal are used as input for the generation of the multimedia effect. According to an aspect the modification data is used to intentionally modify the real-time physiological when generating the multimedia effect. In one example the modification data is used to modify a real-time physiological data, e.g. breathing, so that when outputting the multimedia effect to the person 1 via the multimedia interface 50 of the device, there is no mirroring of the breathing data but e.g. the multimedia effect is mirroring e.g. a lower breathing frequency compared to the actual breathing of the user. In one example this multimedia effect is desired for e.g. supporting the person 1 to calm down and to use the multimedia effect to create a peaceful emotional bond between a person 1 and a device.

According to an aspect the modification data is dependent in the time of day, calendar entries, ambient temperature, a vehicle speed etc. In one example a vehicle speed is above a certain predefined limit that triggers a modification data to e.g. manipulate the actual heartbeat of the person 1 so that the outputted multimedia effect is an effect that is not mirroring the actual heartbeat of the person 1 but instead outputting a multimedia effect as if the actual heartbeat of the person 1 was lower.

According to an aspect the method further comprising the step S6 of transitioning the simulated multimedia effect into a real-time multimedia effect based on a detected real-time physiological data. This means that e.g. in the case that the detection of the real-time physiological data has been absent but is re-established, then the simulation of the multimedia effect can be ceased and instead the real-time physiological data resumes to be the input for generating the multimedia effect. In one example the ongoing simulation of the multimedia effect is compared with the re-established actual real-time physiological data and the simulated multimedia effect is slowly adapted to mirror the actual real-time physiological data of the person 1. According to an aspect transitioning the simulated multimedia effect into a real-time multimedia effect comprising combining the multimedia effect generated based on the real-time physiological data with the simulated multimedia effect.

According to an aspect the multimedia effect is at least one of a visual stimulus effect; a haptic stimulus effect; or an audio stimulus effect. In other words, the multimedia effect can be observed using at least one of the person's eyes, feel or ears. According to an aspect the multimedia interface 50a, 50b, 50c, 50d, ..., 50n is at least one of a visual stimulus device; a haptic stimulus device or an audio stimulus device. In other words, a plurality of different devices can be used for establishing the linkage effect that connects the person 1 with the device in a natural way.

According to an aspect the physiological data is at least one of heartbeat data; breathing data; eye movement data; eye blinking data; eye pupil size data; eye gaze data; skin conductance data; muscle tension data or body temperature data. This means that a plural of physiological data of the person 1 can be used for generating the multimedia effect.

According to an aspect the physiological data is breathing data and the multimedia effect is a pulsing light that is outputted via a light source configured to alternate an intensity and/or a frequency of the light for visualizing the actual breathing of the person 1. According to an aspect the light source is integrated in a button configured to start the vehicle. In other words, the person 1 in the vehicle can see the multimedia effect by observing the pulsing light and recognize the multimedia effect as the common linkage effect that connects the person 1 with the device in a natural way.

According to an aspect the sensor 10a, 10b, 10c, 10d,..., 10n is any of a heartbeat sensor for measuring the pulse of the person 1; a respiration sensor for measuring the breathing of the person 1; a camera for capturing images and video of the person 1; a microphone for recording sound of the person 1; or a pressure sensor for measuring a force generated by the person 1. In other words, different sensors can be used for detecting the real-time physiological data of a person 1.

The user interface system 10 is configured to carry out the described method and any aspect of the method. The processing circuitry 102 of the interface system 100 is further configured to carry out the following aspects of the method:
According to an aspect the processing circuitry 102 is further configured to cause the user interface system 100 to adjust the multimedia effect continuously in real-time based on a change in the detected real-time physiological data. This means that the multimedia effect is constantly mirroring the person's physiological data. According to an aspect the processing circuitry 102 is further configured to cause the user interface system 100 to simulate a multimedia effect based on previous real-time physiological data. In other words, the multimedia effect can be maintained similar as the last detected real-time physiological data. Hence, the multimedia effect may be simulated in order to maintain the common linkage between the person 1 and the device even if the detection of the real-time physiological data of the person 1 is absent.

According to an aspect the processing circuitry 102 is further configured to cause the user interface system 100 to generate a control signal based on the real-time physiological data and a modification data and generate a multimedia effect based on the real-time physiological data and the control signal. This means that the multimedia effect can be generated not only based on the real-time physiological data but also based on a modification signal that affects the generation of the multimedia effect so that both the real-time physiological data and the modification signal are used as input for the generation of the multimedia effect.

An example use case, illustrated by the Fig 1 can be described as the following: A person 1 is entering into a vehicle. The person 1 is sitting in the vehicle seat 25 with the respiration sensor 10a. The user interface system 100 is hence, as described in step S1 detecting the breathing of the person 1 as the real-time physiological data of the person 1. The real-time physiological data, i.e. the breathing of the person 1, is used for generating a multimedia effect as described in the step S2. In this example use case, the multimedia effect is outputted via a multimedia interface 50a in form of pulsing light of a start button, for starting the vehicle, as described in the step S3. The pulsing light of the start button is pulsing with the same frequency as the breathing of the person 1 which creates a common linkage effect that connects the person 1 with the vehicle in a natural way.

The disclosure further proposes a computer program product comprising a non-transitory computer readable medium, having thereon a computer program comprising program instructions, the computer program being loadable into a processing circuitry 102 and configured to cause execution of the method according to any of claims 1 through 10 when the computer program is run by the processing circuitry 102.

The different aspects of the disclosure can be combined with one or more of the other different aspects. In the drawings and specification, there have been disclosed exemplary embodiments. However, many variations and modifications can be made to these embodiments. Accordingly, although specific terms are employed, they are used in a generic and descriptive sense only and not for purposes of limitation, the scope of the embodiments being defined by the following claims.

## Claims

1. A method of generating a multimedia effect for creating an emotional bond between a person (1) and a device, the method comprising:
- (S1) detecting, by a sensor (10a, 10b, 10c, 10d,..., 10n), a real-time physiological data of a person (1);
- (S2) generating a multimedia effect based on the real-time physiological data; and
- (S3) outputting the multimedia effect to the person (1) via a multimedia interface (50a, 50b, 50c, 50d, ..., 50n) of the device.

2. The method according to claim 1 comprising :
- (S4) adjusting the multimedia effect continuously in real-time based on a change in the detected real-time physiological data.

3. The method according to any of the preceding claims comprising :
- (S5) simulating a multimedia effect based on previous real-time physiological data.

4. The method according to any of the preceding claims comprising :
- (S2a) generating a control signal based on the real-time physiological data and a modification data; and
- (S2b) generating the multimedia effect based on the real-time physiological data and the control signal.

5. The method according to claim 3, further comprising :
- (S6) transitioning the simulated multimedia effect into a real-time multimedia effect based on a detected real-time physiological data.

6. The method according to any of the preceding claims, wherein the multimedia effect is at least one of a visual stimulus effect; a haptic stimulus effect; or an audio stimulus effect.

7. The method according to any of the preceding claims, wherein the multimedia interface (50a, 50b, 50c, 50d, ..., 50n) is at least one of a visual stimulus device; a haptic stimulus device or an audio stimulus device.

8. The method according any of the preceding claims, wherein the physiological data is at least one of heartbeat data; breathing data; eye movement data; eye blinking data; eye pupil size data; eye gaze data; skin conductance data; muscle tension data or body temperature data.

9. The method according to any of the preceding claims, wherein the physiological data is breathing data and the multimedia effect is a pulsing light that is outputted via a light source configured to alternate an intensity and/or a frequency of the light for visualizing the actual breathing of the person (1).

10. The method according to any of the preceding claims, wherein the sensor (10a, 10b, 10c, 10d,..., 10n)is any of:
- a heartbeat sensor for measuring the pulse of the person (1);
- a respiration sensor for measuring the breathing of the person (1);
- a camera for capturing images and video of the person (1);
- a microphone for recording sound of the person (1); or
- a pressure sensor for measuring a force generated by the person (1).

11. A user interface system (100) in a vehicle for creating an emotional bond between a person (1) and the vehicle, the user interface system (100) comprising:
• a sensor (10a, 10b, 10c, 10d,..., 10n)configured to detect a real-time physiological data of a person (1);
• a multimedia interface (50a, 50b, 50c, 50d, ..., 50n) configured to output multimedia;
• a processing circuitry (102) configured to cause the user interface system (100) to:
- detect, by the sensor (10a, 10b, 10c, 10d,..., 10n), a real-time physiological data of a person (1);
- generate a multimedia effect based on the real-time physiological data; and
- output the multimedia effect to the person (1) via a multimedia interface (50a, 50b, 50c, 50d, ..., 50n) of the device.

12. The user interface system (100) according to claim 11 wherein the processing circuitry (102) is further configured to cause the user interface system (100) to:
- adjust the multimedia effect continuously in real-time based on a change in the detected real-time physiological data.

13. The user interface system (100) according to claim 11 wherein the processing circuitry (102) is further configured to cause the user interface system (100) to:
- simulate a multimedia effect based on previous real-time physiological data.

14. The user interface system (100) according to claim 11 wherein the processing circuitry (102) is further configured to cause the user interface system (100) to:
- generate a control signal based on the real-time physiological data and a modification data; and
- generate a multimedia effect based on the real-time physiological data and the control signal.

15. The user interface system (100) according to claim 11 wherein the visual stimulus device is any of: a display configured to display a graphical user interface; or a light source configured to alternate illumination of light by changing any of an intensity, a color or a frequency of the light.
